# EUROPÄISCHE PATENTSCHRIFT

(11) **EP 0 978 511 B1**
(45) Veröffentlichungstag und Bekanntmachung des Hinweises auf die Patenterteilung: **22.10.2003**
(21) Anmeldenummer: 99114238.1
(22) Anmeldetag: 27.07.1999
(51) Int. Cl.: C07D 251/32, C08G 18/79, C07D 251/34, C08K 5/3492

(54) **N,N'-disubstituierte N-(2-hydroxyalkyl)-Harnstoffe**
N,N'-disubstituted N-(2-hydroxyalkyl)-Ureas
Urées -(2-hydroxyalkyle) N,N'-disubstituées

(30) Priorität: 07.08.1998 DE 19835703
(43) Veröffentlichungstag der Anmeldung: 09.02.2000
(73) Patentinhaber: Bayer Aktiengesellschaft, 51368 Leverkusen (DE)
(72) Erfinder: Kopp, Richard, Dr., 51061 Köln (DE); Barnes, James-Michael, 53547 Hochscheid (DE); Ruprecht, Hans-Dieter, Dr., 51061 Köln (DE); Wussow, Hans-Georg, Dr., 40597 Düsseldorf (DE)

(56) Entgegenhaltungen:
- DE-A- 2 106 726
- DE-A- 19 611 849

## Beschreibung

Gegenstand der vorliegenden Patentanmeldung sind nichtkristallisierende N,N'-disubstituierte N-(2-hydroxyalkyl)-Harnstoffe sowie deren Verwendung als Hydrolyseschutzmittel in Estergruppen-enhaltenden Kunststoffen.

Aus DE-A 2 106 726 sind N,N'-disubstituierte N-(2-hydroxyalkyl)-Harnstoffe bzw. -Thioharnstoffe bekannt, die als Hydrolyseschutzmittel für Estergruppen enthaltende Kunststoffe eingesetzt werden. Nachteilig bei den in der genannten deutschen Offenlegungsschrift beschriebenen Harnstoffen bzw. Thioharnstoffen ist deren Kristallinität, so daß diese Harnstoffe nur schwer in homogener Form den zu stabilisierenden Estergruppen-haltigen Kunststoffen unterzumischen sind und daher vor der Verarbeitung aufgeschmolzen werden müssen, was einen zusätzlichen Arbeitsschritt beinhaltet. Dieser zusätzliche Arbeitsschritt und die Kristallinität der Hydrolyseschutzmittel geht zu Lasten der Wirtschaftlichkeit bei der Verwendung der in der genannten deutschen Offenlegungsschrift beschriebenen Harnstoffe bzw. Thioharnstoffe für die Stabilisierung von Estergruppen-haltigen Kunststoffen.

Weiterhin ist nachteilig bei der Verwendung der in der deutschen Offenlegungsschrift beschriebenen Harnstoffe bzw. Thioharnstoffe, daß diese bei einer durch unsachgemäße Verarbeitung verursachten Überhitzung, z.B. bei dem Versuch zur Verflüssigung vor der Zumischung zu den Estergruppen-haltigen Kunststoffen, die in der Praxis nicht selten bei stark erhöhten Ofentemperaturen durchgeführt wird, thermisch gespalten werden können, wodurch dann flüchtige Iso(thio)cyanate gebildet werden, die für den Verarbeiter ein physiologisches Risiko darstellen. Eine solche Überhitzung kann auch auftreten beim Einsatz in Systemen, deren Aushärtung bei höheren Temperaturen erfolgt, wie z.B. bei der Schneckenverarbeitung von Thermoplasten oder bei der Aushärtung von Gießelastomeren.

Es wurde nun gefunden, daß beim Einsatz von trimerisiertem Hexamethylendiisocyanat als Ausgangskomponente zur Herstellung von N,N'-disubstituierten N-(2-hydroxyalkyl)-Harnstoffen nichtkristallisierende Produkte erhalten werden, die ohne Probleme, gegebenenfalls als stabile Lösungen in Butandiol-1,4, eingesetzt werden können und einen ausgezeichneten Hydrolyseschutz für Estergruppen-haltige Kunststoffe darstellen.

Gegenstand der vorliegenden Erfindung sind daher nichtkristallisierende N,N'-disubstituierte N-(2-hydroxyalkyl)-Harnstoffe der allgemeinen Formel (I)

R¹-(-NH-CO-NR²-CH₂-CHR³-OH)ₙ (I),

worin
- R¹: für den um die freien NCO-Gruppen verminderten Rest eines trimerisierten Hexamethylendiisocyanates steht,
- R²: einen gegebenenfalls durch Hydroxyl- oder Cyanogruppen oder durch Halogenatome substituierten aliphatischen oder aromatischen Kohlenwasserstoffrest mit bis zu 18 Kohlenstoffatomen darstellt,
- R³: für Wasserstoff oder einen Alkylrest mit 1 bis 12 C-Atomen, bevorzugt 1 bis 3 C-Atomen, steht
und
- n: im Mittel eine Zahl von 3,0 bis 5,0, bevorzugt von 3,2 bis 4,5 bedeutet.

Vorzugsweise werden N,N'-disubstituierte N-(2-hydroxyalkyl)-Harnstoffe der allgemeinen Formel (I) genannt, in welchen
- n: für 3,2 bis 4,5 steht,
- R²: für Methyl oder Hydroxyethyl steht,
- R³: Wasserstoff oder eine Methylgruppe bedeutet und
- R¹: einen um die freien NCO-Gruppen verminderten Rest eines trimerisierten Hexamethylendiisocyanates mit der idealisierten Formel (II) darstellt:

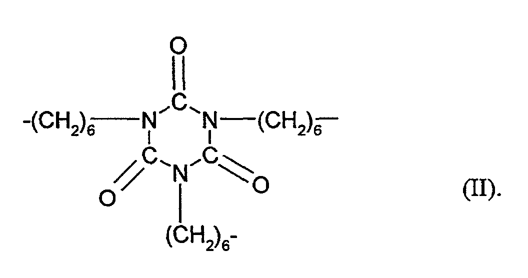

Der Ausdruck "idealisierte Form des trimerisierten Hexamethylendiisocyanates" bezieht sich darauf, daß bei der Herstellung auch Oligomere gebildet werden, die aus über Hexamethylenbrücken verknüpften Trimeren bestehen. Außerdem können die Verbindungen auch sogenannte asymmetrische Trimerenringe der nachstehenden Formel (III) enthalten

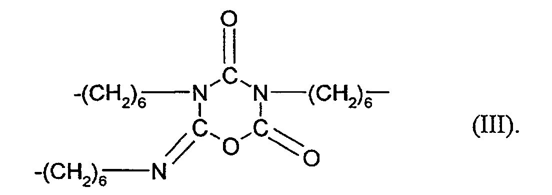

Die Herstellung von Trimeren mit einem erhöhten Anteil dieser asymmetrischen Trimerenringe wird z.B. in DE-A 1 96 11 849 beschrieben.

Die Herstellung der erfindungsgemäßen, nichtkristallisierenden N-Alkyl-N-2-hydroxyalkyl-Harnstoffe der o.a. allgemeinen Formel (I) erfolgt nach bekannten Verfahren der präparativen organischen Chemie durch Addition von N-substituierten-2-Hydroxyalkylaminen an trimerisiertes Hexamethylendiisocyanat bei Reaktionstemperaturen von 20 bis 100°C, bevorzugt von 20 bis 70°C.

Diese Additionsreaktion kann sowohl in Lösung als auch in Substanz durchgeführt werden, erfolgt jedoch bevorzugt in Lösung. Geeignete Lösungsmittel sind z.B. solche, die den Einsatz der erfindungsgemäßen Harnstoffe als Hydrolyseschutzmittel in Kunststoffen nicht negativ beeinflussen, wie übliche Weichmacher, z.B. Dioctylphthalat, oder organische Lösungsmittel wie Ethanol, Isopropanol, Dioxan, Tetrahydrofuran, Chloroform und/oder 1,4-Butandiol.

Zur Entfernung von gegebenenfalls vorhandenen, mit den Isocyanatgruppen nicht oder nur langsam reagierenden Nebenkomponenten der eingesetzten N-substituierten 2-Hydroxyalkylamine, wie z.B. N,N-Dimethylaminoethanol, N,N-Diethylaminoethanol oder Trimethylamin, kann das Umsetzungsprodukt aus der Additionsreaktion, bzw. dessen Lösung, einer kurzen Vakuumbehandlung unterzogen werden, wobei zur Unterstützung der Verflüchtigung zusätzlich ein gelinder Stickstoffstrom durchgeleitet werden kann.

Die Ausgangskomponenten, das sind die substituierten Hydroxylamine sowie das trimerisierte Hexamethylendiisocyanat, werden üblicherweise in einem Äquivalentverhältnis von Isocyanat- zu -NH-Gruppen von 1:1 bis 1,1:1, vorzugsweise von 1:1 bis 1,05:1 eingesetzt.

Als Ausgangsisocyanat zur Herstellung der erfindungsgemäßen Harnstoffe der allgemeinen Formel (I) wird -wie zuvor erwähnt- trimerisiertes Hexamethylendiisocyanat eingesetzt. Dieses erhält man durch unterschüssiges Trimerisieren von Hexamethylendiisocyanat und anschließendes Entfernen des nichtumgesetzten monomeren Hexamethylendiisocyanates. Die Herstellung von trimerisiertem Hexamethylendiisocyanat ist beispielsweise beschrieben in J. Prakt. Chem. 336 (1994) 185-200 oder den EP-A's 339 396 und 798 299. Diese Isocyanate sind als Handelsprodukt erhältlich unter dem Handelsnamen ®Desmodur N 3300 und ®VP LS 2025/1 der Fa. Bayer AG, Leverkusen.

Geeignete Reaktionspartner für das trimerisierte Hexamethylendiisocyanat sind beliebige N-substituierte 2-Hydroxylalkylamine, vorzugsweise 2-Hydroxyethylamine, wie N-Methyl-2-hydroxyethylamin, N-Methyl-2-hydroxybutylamin, N-Isopropyl-2-hydroxyethylamin, N-Butyl-2-hydroxyhexylamin, Bis(2-hydroxyethyl)amin.

Ganz besonders bevorzugt wird N-Methyl-2-hydroxyethylamin eingesetzt. Die N-substituierten 2-Hydroxylalkylamine sind ebenfalls näher beschrieben in der o.g. deutschen Offenlegungsschrift.

Ein weiterer Gegenstand der vorliegenden Erfindung ist die Verwendung der erfindungsgemäßen, nichtkristallisierenden N-Alkyl-N-2-hydroxyalkyl-Harnstoffe der allgemeinen Formel (I) zur Stabilisierung (Hydrolyseschutzmittel) beliebiger Estergruppen-aufweisender Kunststoffe, vorzugsweise Polyurethane.

Im allgemeinen genügen zur ausreichenden Stabilisierung der Estergruppen-aufweisenden Kunststoffe 0,02 bis 10 Gew.-%, bevorzugt 0,1 bis 5 Gew.-%, der erfindungsgemäßen Hydrolyseschutzmittel. Die Hydrolyseschutzmittel der genannten Art können den zu stabilisierenden Kunststoffen entweder nach deren Herstellung zugesetzt werden oder auch bereits bei der Herstellung der Estergruppen-aufweisenden Kunststoffe den für die Herstellung erforderlichen Ausgangskomponenten beigemischt werden. Diese letztgenannte Art der Zugabe empfiehlt sich insbesondere bei der Herstellung von stabilisierten Estergruppen-aufweisenden Polyurethanen. Dabei kann das Hydrolyseschutzmittel entweder der Polyisocyanat- oder der Polyolkomponente zugegeben werden. Unter Estergruppen-aufweisenden Polyurethanen sind beliebige, unter Mitverwendung von Estergruppen-aufweisenden Hydroxylverbindungen hergestellte Polyurethane zu verstehen, z.B. Polyurethan-(Integral)-Schaumstoffe, Polyurethan-Elastomere, thermoplastisches Polyurethan (TPU), Polyurethanfasern sowie Polyurethan-Beschichtungsmittel.

Außer zur Stabilisierung der Estergruppen-aufweisenden Polyurethan-Kunststoffe eignen sich die erfindungsgemäßen Harnstoffe der allgemeinen Formel (I) auch zur Stabilisierung beliebiger anderer Estergruppen-aufweisender Kunststoffe, wie Polyestern aus zweibasischen Dicarbonsäuren, z.B. Phthalsäure, Terephthalsäure und Adipinsäure, und mehrwertigen Alkoholen, wie Ethylenglycol, Hexandiol, Glycerin, Trimethylpropan und Pentaerythrit. Weiterhin sind zu nennen seitenständige Estergruppen-aufweisende Polymere bzw. Copolymere basierend auf Acrylsäure- oder Methacrylsäureester, Vinylester, wie Polyvinylacetat. Derartige Kunststoffe können beispielsweise als Lacke, Folien, Überzüge, Fasern, Schaumstoffe, Elastomere, Gießharze oder als Presskörper vorliegen.

### Beispiele

### Beispiel 1

### Umsetzung von N-Methyl-ethanolamin (N-MEA) mit Hexamethylen-1,6-diiso cyanat (HDI) und trimerisiertem Hexamethylendiisocyanat (N 3300) in Isopropanol

Im Beispiel 1 werden die N-Methyl-N-hydroxyethylharnstoffe auf Basis von HDI und auf Basis von trimerisiertem HDI (N 3300) in Isopropanol hergestellt und anschließend die Eigenschaften der Lösungen und der isolierten Harnstoffe ermittelt.

| **Versuch** | **Isocyanat** | **NCO- Gehalt** **[Gew.-%]** | **Einsatzmenge** **[g]** | **Äquiv. NCO** | **Menge N-MEA** **[g]** | **Äquiv. NH** | **Kennzahl NCO:NH** |
|---|---|---|---|---|---|---|---|
| a | HDI | 49,98 | 60,00 | 0,71 | 53,60 | 0,71 | 1,00 |
| b | N 3300^{a} | 21,30 | 68,97 | 0,35 | 26,30 | 0,35 | 1,00 |

| | | | | | | | |
|---|---|---|---|---|---|---|---|
| a: ®Desmodur N 3300, Bayer AG, Leverkusen | | | | | | | |

### Durchführung:

In einem 250 ml Dreihalskolben werden 100 Gewichtsteile Isopropanol zusammen mit der angegebenen Menge N-Methyl-ethanolamin unter Rühren vorgelegt. Die auf 40°C erwärmte Isocyanatkomponente wird unter Rühren zugetropft, wobei die Reaktionstemperatur durch leichte Kühlung auf 50°C begrenzt wird.

Nach beendetem Zutropfen wird so lange weitergerührt, bis die typische Bande der Isocyanatgruppe bei ca. 2 250 cm⁻¹ im Spektrum nicht mehr zu sehen ist.

### Ergebnis:

Beim Versuch a wird zunächst eine bei 50°C homogene Lösung erhalten, die nach Abkühlen und Stehen bei Raumtemperatur plötzlich spontan auskristallisiert. Beim Versuch b wird eine klare Lösung mit einer Viskosität von 390 mPa.s bei 25°C erhalten, die sich bei längerem Stehen nicht verändert.

Eine Teilmenge der erhaltenen Produkte wird am Rotationsverdampfer im Wasserstrahlvakuum vorsichtig eingedampft. Beim Ansatz a erhält man weiße Kristalle mit einem Schmelzpunkt von ca. 97°C (Koflerbank), beim Ansatz b eine viskose Flüssigkeit, die auch beim Abkühlen auf 0°C nicht kristalisiert.

### Beispiel 2

### Umsetzung von N-Methyl-ethanolamin (N-MEA) mit Hexamethylen-1,6-diisocyanat (HDI) und trimerisiertem Hexamethylendiisocyanat (N 3300) in Butandiol-1,4

Die Einsatzmengen sind so gewählt, daß beide Produkte die gleiche Konzentration an den für den Hydrolyseschutz ausschlaggebenden N-Methyl-N-hydroxyethyl-Gruppen, berechnet als Gehalt an N-MEA, aufweisen.

| **Versuch** | **Isocyanat** | **NCO-Gehalt** **[Gew.-%]** | **Einsatzmenge** **[g]** | **Äquiv. NCO** | **Menge N-MEA** **[g]** | **Äquiv . NH** | **Menge Butan- diol-1,4** **[g]** | **Kennzahl NCO:NH** | **Gehalt N-MEA** **[Gew.-%]** |
|---|---|---|---|---|---|---|---|---|---|
| a | HDI | 49,98 | 207,40 | 2,47 | 176,40 | 2,35 | 575,00 | 1,05 | 18,40 |
| b | N 3300 | 21,63 | 476,34 | 2,45 | 175,44 | 2,34 | 301,92 | 1,05 | 18,40 |

### Durchführung:

In einem 2 Dreihalskolben werden Butandiol-1,4 und N-Methyl-ethanolamin unter Rühren vorgelegt. Die auf 40°C erwärmte Isocyanatkomponente wird unter Rühren zugetropft, wobei die Reaktionstemperatur durch leichte Kühlung auf ca. 50°C begrenzt wird.

Nach beendetem Zutropfen wird so lange weitergerührt, bis die typische Bande der Isocyanatgruppe bei ca. 2 250 cm⁻¹ im IR-Spektrum nicht mehr zu sehen ist.

### Ergebnis:

Beim Versuch a wird zunächst eine klare, homogene Lösung erhalten, in der sich beim Abkühlen auf Raumtemperatur allmählich Kristalle bilden. Nach einiger Zeit bei Raumtemperatur ist die Mischung fast vollständig durchkristallisiert. Durch Erhitzen auf 55 bis 50°C kann der Kristallbrei wieder aufgeschmolzen werden.

Beim Versuch b wird eine homogene Lösung mit einer Viskosität von 3 340 mPa.s bei 25°C erhalten, die beim Abkühlen auf 0°C noch fließfähig ist und keinerlei Tendenz zum Kritallisieren zeigt.

### Beispiel 3

### Wirksamkeit der erfindungsgemäßen Hydrolysestabilisatoren in PUR-Gießsystemen

### Beispiel 3a (Vergleichsbeispiel, unstabilisiert)

1 000 g (0,5 mol) eines handelsüblichen Polyethylenadipats mit einem mittleren Molgewicht von 2 000 wurden auf 125°C erwärmt, mit 180 g (1,714 mol) Naphthylen-1,5-diisocyanat versetzt, sofort intensiv verrührt und nach 2 Minuten im Vakuum entgast. In einer exothermen Reaktion entstand nach ca. 15 Minuten ein NCO-Prepolymer. In dieses Prepolymer wurden als Vernetzer 20 g (0,222 mol) 1,4-Butandiol eingerührt. Das reagierende Gemisch wurde innerhalb von 1 Minute in auf 110°C vorgewärmte Formen gegossen und erstarrte darin nach wenigen Minuten. Das resultierende Elastomer wurde 24 Stunden bei 110°C nachgetempert und anschließend 4 Wochen bei Raumtemperatur gelagert.

Es entstand ein Elastomer mit den in Tabelle 1 wiedergegebenen Eigenschaften. Die Hydrolysenanfälligkeit dieses Elastomers wird ebenfalls in Tabelle 1 dokumentiert.

### Beispiel 3b (erfindungsgemäß)

Es wurde analog Vergleichsbeispiel 3a ein Prepolymer hergestellt. Abweichend von Vergleichsbeispiel 3a verwendete man als Vernetzer eine Mischung aus 1,4-Butandiol (16,11 g) und der im Beispiel 2b hergestellten 1,4-Butandiol-haltigen Hydrolysenschutzmittellösung (12,5 g). Das resultierende Elastomer wurde 24 Stunden bei 110°C nachgetempert und anschließend 4 Wochen bei Raumtemperatur gelagert.

Es entstand ein Elastomer mit den in Tabelle 1 wiedergegebenen Eigenschaften. Die verbesserte Hydrolysenbeständigkeit dieses Elastomers im Vergleich zum Beispiel 3a wird ebenfalls in Tabelle 1 dokumentiert.

**Tabelle 1**

| **Pysikalische Prüfwerte Physical properties** | | **DIN** **ISO** | **Beispiel 3a** **(Vergleich)** | **Beispiel 3b** **(erf.gemäß)** |
|---|---|---|---|---|
| Spannung bei 100% Dehnung | | 53504 | 4,3 | 4,2 |
| Stress at 100% strain | (MPa) | 37 | | |
| Spannung bei 300% Dehnung | | 53504 | 7,8 | 7,5 |
| Stress at 300% strain | (MPa) | 37 | | |
| Reißfestigkeit | | 53504 | 49 | 39 |
| Tensile strength | (MPa) | 37 | | |
| Reißdehnung | | 53504 | 663 | 632 |
| Elongation at break | (%) | 37 | | |
| Weiterreißwiderstand (Graves) | | 53515 | 31 | 27 |
| Tear propagation resistance | (kN/m) | 34 | | |
| Rückprallelastizität | | 53512 | 60 | 61 |
| Rebound resilience | (%) | 4662 | | |
| Abriebverlust | | 53516 | 37 | 51 |
| Abrasion loss | (mm³) | 4649 | | |
| Druckverformungsrest | | 53517 | 18 | 26 |
| Compression set | | 815-1991 | | |
| 70h/23°C | (%) | | | |

| **Eigenschaften nach 11d** **Wasseralterung bei 80°C** | | | | |
|---|---|---|---|---|
| Spannung bei 100% Dehnung | | 53504 | zerstört | 2,7 |
| Stress at 100% strain | (MPa) | 37 | | |
| Spannung bei 300% Dehnung | | 53504 | | 4,3 |
| Stress at 300% strain | (MPa) | 37 | | |
| Reißfestigkeit | | 53504 | | 16,4 |
| Tensile strenght | (MPa) | 37 | | |
| Reißdehnung | | 53504 | | 1082 |
| Elongation at break | (%) | 37 | | |

### Beispiel 4

### Vergleich handelsüblicher Hydrolyseschutzmittel mit dem Produkt aus Beispiel 2b

### Rezeptur:

| **Versuch** | **a** | **b** | **c** | **d** | **e** | **f** | **g** |
|---|---|---|---|---|---|---|---|
| PE 225 B^{a} | 100,0 | 100,0 | 100,0 | 100,0 | 100,0 | 100,0 | 100,0 |
| 1,4-Butandiol | 10,5 | 10,5 | 10,5 | 10,5 | 10,5 | 10,5 | 10,5 |
| Loxamid EBS^{b} | 0,5 | 0,5 | 0,5 | 0,5 | 0,5 | 0,5 | 0,5 |
| Stabaxol 1^{c} | - | 0,4 | 0,8 | - | - | - | - |
| Stabaxol P 200^{d} | - | - | - | 0,4 | 0,8 | - | - |
| Beispiel 2b | - | - | - | - | - | 0,4 | 0,8 |
| | Vergleich | | | | | erfindungsgemäß | |

MDI Kennzahl: 1.01

### Ausgangsprodukte:

a: Polyester aus Adipinsäure und Butandiol-1,4, MG 2250, OH-Zahl 50
b: Wachs; Hersteller Fa. Henkel KGaA
c: Hydrolysestabilisator vom Carbodiimid-Typ, Fa.Rheinchemie, Mannheim
d: Hydrolysestabilisator vom Carbodiimid-Typ, Fa. Rheinchemie, Mannheim

### Gießversuche:

Die Gießversuche wurden nach dem One-Shot-Verfahren durchgeführt. Stabaxol 1, Stabaxol P 200 oder das Produkt aus Beispiel 2b und Ester ließ man über Nacht miteinander reagieren.

Nach Zusammengabe von Stabilisator-haltigem Ester mit dem Loxamid EBS und 1,4-Butandiol wurde die Temperatur mittels einer Gaskochstelle auf 140°C gebracht. Danach wurde das MDI unter Rühren dazugegeben (es wurde ca. 30 sec eingerührt). Dann wurde das Reaktionsprodukt auf ein auf 80°C vorgeheiztes Blech gegossen, 5 min stehengelassen, 30 min im Trockenschrank bei 80°C nachgeheizt und anschließend über Nacht abkühlen gelassen. Anschließend wurden die Platten geschnitten und granuliert.

### Verarbeitung:

Die Granulatgemische wurden getrocknet und dann auf einer Spritzgußmaschine des Typs Mannesmann-Demag D60 zu Prüfkörpern verspritzt. Nach dem Tempern wurden die Spritzkörper zur Prüfung gegeben.

Der erfindungsgemäße Stabilisator aus Beispiel 2b zeigt im Rahmen der Messgenauigkeit eine den handelsüblichen Stabilisatoren vom Carbodiimid-Typ vergleichbare Wirksamkeit.

**Tabelle 2**

| **Versuch** | **a** | **b** | **c** | **d** | **e** | **f** | **g** |
|---|---|---|---|---|---|---|---|
| **Stabilisator** | ohne | Stabaxol 1 | | Stabaxol P 200 | | Verbindung aus Beispiel 2b | |
| **O-Wert** | | | | | | | |
| Spannung 100% (N/mm²) | 5,9 | 5,8 | 5,8 | 5,8 | 5,5 | 6,1 | 5,6 |
| Spannung 300% (N/mm²) | 15,2 | 16,6 | 15,3 | 16,9 | 15,0 | 17,2 | 14,8 |
| Reißfestigkeit (N/mm²) | 58,4 | 50,5 | 52,1 | 54,6 | 53,5 | 54,6 | 56,3 |
| Reißdehnung (%) | 540 | 507 | 553 | 515 | 539 | 508 | 562 |
| E-Modul Zug | 17,4 | 16,2 | 20,0 | 18,7 | 12,5 | 17,3 | 18,3 |

| **7d-Wert** | | | | | | | |
|---|---|---|---|---|---|---|---|
| Spannung 100% (N/mm²) | 5,0 | 5,3 | 5,4 | 5,3 | 5,5 | 5,6 | 5,2 |
| Spannung 300% (N/mm²) | 12,1 | 13,7 | 13,4 | 14,3 | 15,1 | 15,2 | 13,2 |
| Reißfestigkeit (N/mm²) | 49,3 | 47,9 | 50,1 | 50,2 | 49,3 | 48,0 | 52,1 |
| Reißdehnung (%) | 630 | 533 | 547 | 525 | 519 | 515 | 602 |
| E-Modul Zug | 14,8 | 16,2 | 17,4 | 13,5 | 14,7 | 16,1 | 19,7 |

| **14d-Wert** | | | | | | | |
|---|---|---|---|---|---|---|---|
| Spannung 100% (N/mm²) | 4,1 | 5,2 | 4,8 | 4,8 | 5,0 | 5,1 | 4,9 |
| Spannung 300% (N/mm²) | 8,3 | 13,3 | 12,3 | 12,6 | 13,5 | 13,6 | 12,3 |
| Reißfestigkeit (N/mm²) | 19,1 | 49,5 | 46,7 | 47,9 | 49,7 | 47,0 | 47,2 |
| Reißdehnung (%) | 652 | 650 | 590 | 577 | 442 | 561 | 626 |
| E-Modul Zug | 13,7 | 14,8 | 18,6 | 16,1 | 18,8 | 12,5 | 16,1 |

| **21d-Wert** | | | | | | | |
|---|---|---|---|---|---|---|---|
| Spannung 100% (N/mm²) | - | 4,9 | 4,8 | 4,7 | 5,1 | 5,1 | 4,8 |
| Spannung 300% (N/mm²) | - | 12,5 | 11,5 | 11,5 | 13,3 | 13,2 | 11,9 |
| Reißfestigkeit (N/mm²) | 1,7 | 45,3 | 42,5 | 41,0 | 44,1 | 42,5 | 46,0 |
| Reißdehnung (%) | 25,5 | 560 | 600 | 626 | 528 | 548 | 622 |
| E-Modul Zug | 10,0 | 14,8 | 13,5 | 11,3 | 12,5 | 16,6 | 16,1 |

## Patentansprüche

1. Nichtkristallisierende N,N'-disubstituierte N-(2-hydroxyalkyl)-Harnstoffe der allgemeinen Formel (I)
R¹-(-NH-CO-NR²-CH₂-CHR³-OH)ₙ (I),
worin
R¹ für den um die freien NCO-Gruppen verminderten Rest eines trimerisierten Hexamethylendiisocyanates steht,
R² einen gegebenenfalls durch Hydroxyl- oder Cyanogruppen oder durch Halogenatome substituierten aliphatischen oder aromatischen Kohlenwasserstoffrest mit bis zu 18 Kohlenstoffatomen darstellt,
R³ für Wasserstoff oder einen Alkylrest mit 1 bis 12 C-Atomen steht
und
n im Mittel eine Zahl von 3,0 bis 5,0 bedeutet.

2. N-Alkyl-N-2-hydroxyalkyl-Harnstoffe der allgemeinen Formel (I), **dadurch gekennzeichnet, daß**
n für 3,2 bis 4,5 steht,
R² für Methyl oder Hydroxyethyl steht,
R³ Wasserstoff oder eine Methylgruppe bedeutet und
R¹ einen um die freien NCO-Gruppen verminderten Rest eines trimerisierten Hexamethylendiisocyanates mit der idealisierten Formel (II) darstellt:

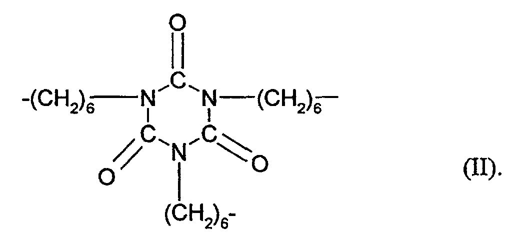

3. Verwendung der Harnstoffe nach Anspruch 1 als Hydrolyseschutzmittel für Estergruppen-enthaltende Kunststoffe.

4. Verwendung der Harnstoffe nach Anspruch 1 als Hydrolyseschutzmittel für Estergruppen-enthaltende Polyurethane.

## Claims

1. Non-crystallising N,N'-disubstituted N-(2-hydroxyalkyl)-ureas of general formula (I)
R¹-(-NH-CO-NR²-CH₂-CHR³-OH)ₙ (I),
wherein
R¹ represents the radical of a trimerised hexamethylene diisocyanate which is diminished by the loss of the free NCO groups,
R² represents an aliphatic or aromatic hydrocarbon radical comprising up to 18 carbon atoms, which is optionally substituted by hydroxyl or cyano groups or by halogen atoms,
R³ represents hydrogen or an alkyl radical comprising 1 to 12 C atoms,
and
n on average denotes a number from 3.0 to 5.0.

2. N-alkyl-N-2-hydroxyalkyl-ureas of general formula (I), **characterised in that**
n represents 3.2 to 4.5,
R² represents methyl or hydroxyethyl,
R³ denotes hydrogen or a methyl group, and
R¹ represents a radical of a trimerised hexamethylene diisocyanate, which is diminished by the loss of the free NCO groups, with the idealised formula (II):

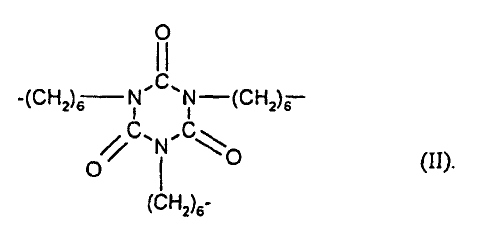

3. Use of the ureas according to claim 1 as hydrolysis protection agents for plastics which contain ester groups.

4. Use of the ureas according to claim 1 as hydrolysis protection agents for polyurethanes which contain ester groups.

## Revendications

1. N-(2-hydroxyalkyl)-urées N,N'-disubstituées ne cristallisant pas de la formule générale (I)
R¹-(-NH-CO-NR²-CH₂-CHR³-OH)ₙ (I),
dans laquelle
R¹ représente le reste réduit autour des groupes libres NCO d'un diisocyanate d'hexaméthylène trimérisé,
R² représente un reste hydrocarboné ayant jusqu'à 18 atomes de carbone, aliphatique ou aromatique, éventuellement substitué par des groupes hydroxyle ou cyano ou par des atomes d'halogène,
R³ représente un atome d'hydrogène ou un reste alkyle avec de 1 à 12 atomes de C,
et
n est en moyenne un nombre de 3,0 à 5,0.

2. N-alkyl-N-2-hydroxyalkyl-urées de la formule générale (I) **caractérisées en ce que**
n est compris entre 3,2 et 4,5,
R² représente le groupe méthyle ou hydroxyéthyle,
R³ représente un atome d'hydrogène ou un groupe méthyle et
R¹ représente un reste réduit autour des groupes NCO libres d'un diisocyanate d'hexaméthylène trimérisé avec la formule idéale (II) :

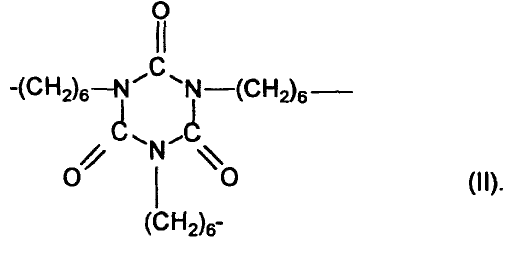

3. Utilisation des urées selon la revendication 1 comme agent de protection à l'hydrolyse pour des matières plastiques contenant des groupes ester.

4. Utilisation des urées selon la revendication 1 comme agent de protection à l'hydrolyse pour des polyuréthannes contenant des groupes ester.
